(19) Europäisches Patentamt
European Patent Office
Office européen des brevets

(11) Numéro de publication : **0 404 640 B1**

# (12) FASCICULE DE BREVET EUROPEEN

(45) Date de publication du fascicule du brevet : **16.02.94 Bulletin 94/07**

(21) Numéro de dépôt : **90401641.7**

(22) Date de dépôt : **13.06.90**

(51) Int. Cl.$^5$ : **C07C 43/23,** A61K 7/48, A61K 31/05, A61K 31/085, A61K 31/12, A61K 31/19, C07C 49/84, C07C 65/26, C07C 43/00, A61K 7/00

## (54) Nouveaux dérivés de tétrahydro-5,6,7,8 naphtalénol-1, leur procédé de préparation et leur utilisation en tant qu'agents antioxydants dans des compositions cosmétiques et pharmaceutiques les contenant.

(30) Priorité : **13.06.89 FR 8907805**

(43) Date de publication de la demande : **27.12.90 Bulletin 90/52**

(45) Mention de la délivrance du brevet : **16.02.94 Bulletin 94/07**

(84) Etats contractants désignés : **AT BE CH DE DK ES FR GB GR IT LI NL SE**

(56) Documents cités :
**EP-A- 0 176 035**
**FR-A- 2 246 528**
**CHEMICAL ABSTRACTS, vol. 32, no. 19, 10 octobre 1938, colonnes 7972-7973, Columbus, Ohio, US; F. VAN WERDER et al.: "Synthetic compounds with vitamin E activity"**
**CHEMICAL ABSTRACTS, vol. 107, no. 17, 26 octobre 1987, page 120, résumé no. 147894f, Columbus, Ohio, US; F. ZOCCARATO et al.: "Inhibition by some phenolic antioxidants of calcium uptake and neurotransmitter release from brain synaptosomes"**

(73) Titulaire : **L'OREAL**
**14, Rue Royale**
**F-75008 Paris (FR)**

(72) Inventeur : **Maignan, Jean**
**8, rue Halévy**
**F-93290 Tremblay les Gonesse (FR)**
Inventeur : **Malle, Gérard**
**18, Grande rue**
**F-77580 Villiers sur Morin (FR)**
Inventeur : **Deflandre, André**
**Route de Manon**
**F-60560 Orry-La-Ville (FR)**
Inventeur : **Lang, Gérard**
**44, avenue Lacour**
**F-95210 Saint Gratien (FR)**

(74) Mandataire : **Stalla-Bourdillon, Bernard et al**
**CABINET NONY & CIE 29, rue Cambacérès**
**F-75008 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux dérivés de tétrahydro-5,6,7,8 naphtalénol-1, leur procédé de préparation et leur utilisation en tant qu'agents antioxydants dans des compositions cosmétiques et dans des compositions pharmaceutiques pour le traitement préventif d'inflammations et allergies cutanées ou de certaines formes de cancers.

Les nouveaux dérivés de tétrahydro-5,6,7,8 naphtalénol-1 selon la présente invention se sont avérés présenter de façon inattendue d'excellentes propriétés antioxydantes vis-à-vis de la peroxydation des lipides polyinsaturés et également vis-à-vis des substances susceptibles de subir des réactions d'oxydation thermo ou photo-induites (telles que des protéines, des sucres, des pigments, des vitamines, des polymères...).

Or, on sait que la peroxydation des lipides implique la formation de radicaux libres intermédiaires qui endommagent les membranes cellulaires se composant, entre autre, de phospholipides et sont responsables notamment des phénomènes du vieillissement de la peau (A.L. TAPPEL dans "Federation Proceedings" vol. 32, n°8, Août 1973).

Par leurs propriétés antioxydantes, les nouveaux dérivés de tétrahydro-5,6,7,8 naphtalénol-1 peuvent donc permettre de mieux lutter contre le vieillissement prématuré de la peau dû à la peroxydation des lipides cutanés.

Ils permettent également d'assurer une meilleure conservation des compositions cosmétiques ou pharmaceutiques comportant une phase grasse en évitant le rancissement des lipides insaturés qui y sont contenus et qui peuvent être d'origine animale comme la lanoline, la cétine (blanc de baleine),la cire d'abeille, le perhydrosqualène, l'huile de tortue, ou d'origine végétale comme l'huile d'olive, l'huile de ricin, l'huile de maïs, l'huile d'amande douce, l'huile d'avocat, l'huile de karité, l'huile de tournesol, l'huile de soja, l'huile d'arachide, les huiles de coprah ou de palmiste hydrogénées, des acides gras essentiels comme la vitamine F et certaines huiles essentielles présentes dans les parfums comme l'huile de citron ou de lavande.

Ces nouveaux dérivés permettent également d'éviter la dégradation oxydative de composés actifs contenus dans des compositions pharmaceutiques (vitamine A, caroténoïde...).

De façon tout à fait surprenante, il a également été constaté que les dérivés de tétrahydro-5,6,7,8 naphtalénol-1, selon la présente invention, pouvaient également être utilisés dans le traitement des inflammations et allergies cutanées et également dans la prévention de certains cancers.

Outre leur bonnes propriétés antioxydantes, les nouveaux dérivés de tétrahydro-5,6,7,8 naphtalénol-1 présentent un excellent caractère liposoluble ainsi qu'une très bonne stabilité thermique. Par ailleurs, ils présentent également l'avantage de ne pas être toxiques ou irritants et d'avoir une parfaite innocuité vis-à-vis de la peau.

Ces nouveaux dérivés de tétrahydro-5,6,7,8 naphtalénol-1 peuvent être représentés par la formule générale suivante:

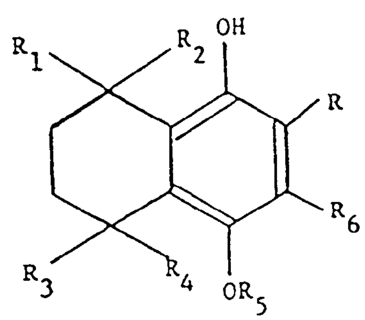

(I)

dans laquelle:

$R_1$, $R_2$, $R_3$ et $R_4$ représentent un radical alkyle inférieur,

$R_5$ et $R_6$ représentent un atome d'hydrogène ou un radical alkyle inférieur,

R représente un atome d'hydrogène, un radical alkyle en $C_1$-$C_{18}$, un radical alkyle en $C_2$-$C_{18}$ substitué par un ou plusieurs hydroxyle(s), un radical alkényle en $C_3$-$C_{18}$, un radical acyle en $C_2$-$C_{18}$, un radical benzyle, un radical benzoyle, un radical carboxyle et les sels carboxyliques d'un métal alcalin ou alcalino-terreux ou d'une amine organique.

Par radical alkyle inférieur, on doit entendre un radical ayant de 1 à 6 atomes de carbone.

Parmi les radicaux alkyles inférieurs et ceux ayant jusqu'à 18 atomes de carbone, on peut notamment citer les radicaux méthyle, éthyle, isopropyle, isobutyle, butyle, tertiobutyle, éthyl-2 hexyle, isooctyle, dodécyle, hexadécyle et octadécyle.

Parmi les radicaux alkyles en $C_2$-$C_{18}$ substitués par un ou plusieurs hydroxyle(s), on peut notamment citer les radicaux hydroxy-1 éthyle, hydroxy-2 propyle, hydroxy-1 propyle, hydroxy-1 butyle, hydroxy-1 hexyle, hydroxy-1 éthyl-1 hexyle ou dihydroxy-2,3 propyle.

Parmi les radicaux alkényles en $C_3$-$C_{18}$ on peut notamment citer les radicaux propényle, butényle, hexényle, octényle, dodécényle et octadécényle.

Parmi les radicaux acyles en $C_2$-$C_{18}$ on peut notamment citer les radicaux acétyle, propionyle, butyryle, isobutyryle, hexanoyle, octanoyle, dodécanoyle et octadécanoyle.

Lorsque R est un radical benzyle ou benzoyle, celui-ci peut être représenté par la formule suivante:

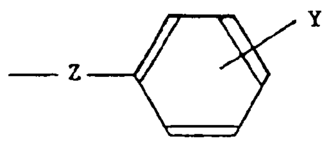

dans laquelle:

Z représente -CO-, -CHOH- ou $-CH_2-$ et

Y représente un atome d'hydrogène, un halogène de préférence le chlore, un alcoxy de préférence méthoxy ou le radical trifluorométhyle.

Les sels des composés de formule (I) lorsque R =-COOH sont de préférence des sels de sodium, potassium, magnésium ou d'une amine organique telle que la triéthanolamine.

Parmi les dérivés de tétrahydro-5,6,7,8 naphtalénol-1 particulièrement préférés correspondant à la formule générale (I) on peut notamment mentionner les suivants:

- Méthoxy-4 tétraméthyl-5,5,8,8 tétrahydro-5,6,7,8 naphtalénol-1,
- Acétyl-2 méthoxy-4 tétraméthyl-5,5,8,8 tétrahydro-5,6,7,8 naphtalénol-1,
- (hydroxy-1 éthyl)-2 méthoxy-4 tétraméthyl-5,5,8,8 tétrahydro-5,6,7,8 naphtalénol-1,
- (hydroxy-2 propyl-2)-2 méthoxy-4 tétraméthyl-5,5,8,8 tétrahydro-5,6,7,8 naphtalénol-1,
- (propényl-2)-2 méthoxy-4 tétraméthyl-5,5,8,8 tétrahydro-5,6,7,8 naphtalénol-1,
- Acide hydroxy-1 méthoxy-4 tétraméthyl-5,5,8,8 tétrahydro-5,6,7,8 naphtalène carboxylique,
- Dihydroxy-1,4 tétraméthyl-5,5,8,8 tétrahydro-5,6,7,8 naphtalène,
- Benzoyl-2 méthoxy-4 tétraméthyl-5,5,8,8 tétrahydro-5,6,7,8 naphtalénol-1,
- Benzyl-2 méthoxy-4 tétraméthyl-5,5,8,8 tétrahydro-5,6,7,8 naphtalénol-1.

La présente invention a également pour objet le procédé de préparation des composés de formule (I) telle que définie ci-dessus.

Ce procédé peut être représenté à l'aide du schéma réactionnel suivant:

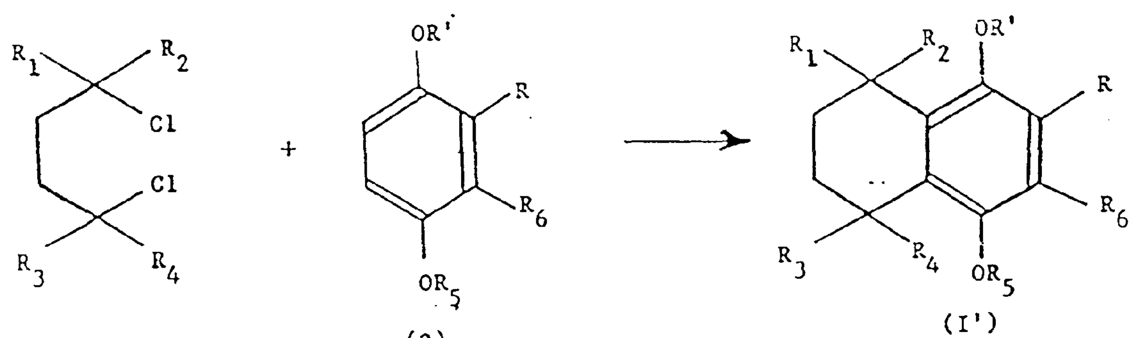

(1) + (2) → (I')

R' = H ou alkyle

Il consiste essentiellement à faire réagir un dichloro-2,5 alcane (1) sur un alcoxy-4 phénol (2) (R'=H) ou un dialcoxy-1,4 benzène (2) (R' = alkyle) dans un solvant organique tel que le nitrométhane ou un solvant dichloré tel que le dichloro-1,2 éthane sou le chlorure de méthylène mais de préférence le dichloro-1,2 éthane en présence d'un acide de Lewis de préférence le chlorure d'aluminium. Cette réaction n'est bien entendu possible que lorsque la nature des substituants R et $R_6$ le permet.

Lorsque le composé de départ (2) est un dialcoxy-1,4 benzène, le dérivé dialcoxy-1,4 de formule (I') (R'=alkyle) est alors soumis à un traitement en présence d'acide iodhydrique ou bromhydrique pour conduire

au phénol correspondant (I') (R'=H).

Selon un mode de réalisation préféré, les composés de formule (I) sont obtenus à partir des composés de formule (I') dans lesquels R = H et ceci selon les schémas réactionnels suivants:

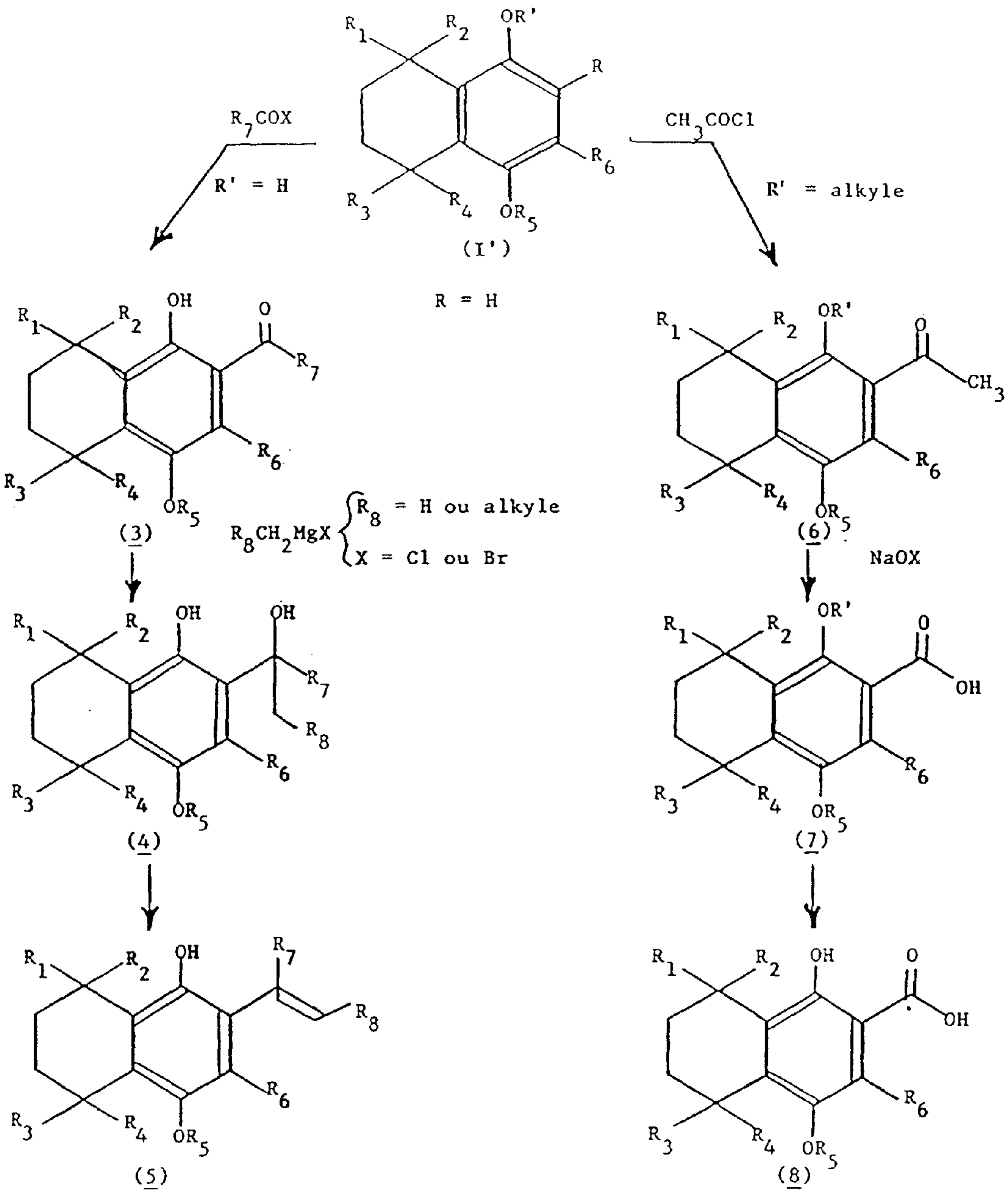


Par acylation du composé de formule (I') dans laquelle R et R' = H à l'aide d'un halogénure d'acide ou d'un anhydride d'acide en présence de chlorure d'aluminium, on obtient la cétone de formule (3) avec un très bon rendement.

Cette cétone (3) peut être réduite par le borohydrure de sodium en alcool correspondant dans un solvant

tel que le méthanol ou l'éthanol ou dans un éther tel que le tétrahydrofuranne ou dans un mélange de ces solvants.

La cétone (3) peut également être transformée en alcool tertiaire (4) par action directe d'un halogénure d'alkyle magnésium dans les conditions classiques des réactions des organo-magnésiens.

Cet alcool tertiaire (4) traité en milieu acide conduit à l'alcène correspondant (5) avec un bon rendement.

Le composé de formule (I') dans laquelle R = H et R' = alkyle peut être acylé par action du chlorure d'acétyle en présence de chlorure d'aluminium pour conduire à la cétone (6).

Celle-ci peut alors être transformée par oxydation à l'hypochlorite ou l'hypobromite de sodium en acide correspondant (7).

Cet acide (7) traité par l'acide iodhydrique ou bromhydrique dans un acide organique tel que l'acide acétique conduit alors à l'acide (8).

Selon la durée du traitement à l'acide iodhydrique ou bromhydrique, cet acide (8) doit être purifié car il contient soit un reste de produit de départ (7), soit un dérivé d'hydroquinone (composé de formule (8) dans laquelle $R_5$ = H).

La présente invention a également pour objet une composition cosmétique comprenant, dans un support cosmétiquement acceptable contenant au moins une phase grasse, une quantité efficace d'au moins un dérivé de tétrahydro-5,6,7,8 naphtalénol-1 de formule (I) telle que définie ci-dessus.

La composition cosmétique de l'invention peut être utilisée comme composition protectrice de l'épiderme humain ou des cheveux ou comme composition anti-solaire.

Selon l'invention le composé de formule (I) est généralement présent dans une proportion comprise entre 0,05 et 10% par rapport au poids total de la composition cosmétique et de préférence entre 0,1 et 5%.

Dans les compositions selon l'invention, le composé de formule (I) agit en tant qu'agent antioxydant. Ces compositions peuvent être des compositions capillaires telles que des laques pour cheveux, des lotions de mise en plis ou éventuellement traitantes ou démêlantes, des shampooings des shampooings colorants, des compositions tinctoriales pour cheveux, des produits de maquillage tels que des vernis à ongles, des crèmes et huiles de traitement pour l'épiderme, des fonds de teint, des bâtons de rouge à lèvres, des compositions pour les soins de la peau telles que des huiles ou crèmes pour le bain ainsi que toute autre composition cosmétique pouvant présenter du fait de ses constituants des problèmes de stabilité à l'oxydation au cours du stockage.

Comme ceci a été précisé ci-dessus, les composés de formule (I) présentent également une activité pharmacologique intéressante dans le domaine du traitement préventif des inflammations et allergies cutanées et peuvent également être utilisés dans la prévention de certains cancers.

L'invention a donc pour objet un composé de formule (I) dans son utilisation comme médicament.

L'invention a également pour objet une composition pharmaceutique, contenant une quantité efficace d'au moins un composé de formule (I) à titre d'ingrédient actif dans un support ou un excipient non toxique.

La composition pharmaceutique conforme à l'invention peut être administrée par voie orale ou par voie topique.

Pour la voie orale, la composition pharmaceutique peut se présenter sous forme de comprimés, de gelules, de dragées, de sirops, de suspensions, de solutions, d'émulsions, etc...

Pour la voie topique, la composition pharmaceutique selon l'invention se présente sous forme d'onguents, de crèmes, de pommades, de solutions, de lotions, de gels, de sprays, de suspensions, etc...

Cette composition médicamenteuse peut contenir des additifs inertes ou pharmacodynamiquement actifs et notamment, des agents hydratants, des antibiotiques, des agents anti-inflammatoires stéroïdiens ou non stéroïdiens, des caroténoïdes, des agents anti-psoriasiques.

Cette composition peut également contenir des agents d'amélioration de la saveur, des agents conservateurs, des agents stabilisants, des agents régulateurs d'humidité, des agents régulateurs de pH, des agents modificateurs de la pression osmotique, des agents émulsionnants, des anésthésiques locaux, des tampons etc....

Elle peut aussi être conditionnée sous des formes retard ou à libération progressive.

Le composé de formule (I) conforme à l'invention est généralement présent dans les compositions pharmaceutiques en une proportion comprise entre 0,01 et 20% en poids par rapport au poids total de la composition et de préférence entre 0,1 et 5% en poids.

Dans l'application thérapeutique, le traitement est déterminé par le praticien et peut varier selon l'âge, le poids et la réponse du patient ainsi que la gravité des symptômes.

Lorsque les composés de formule (I) sont administrés par voie orale, le dosage est généralement compris entre 0,1 et 50mg/kg/jour et de préférence entre 0,2 et 20mg/kg/jour. La durée du traitement est variable selon la gravité des symptômes et peut s'étaler entre 1 et 25 semaines de façon continue ou discontinue.

Les compositions par voie topique contiennent de préférence de 0,25% à 4% en poids du composé de formule (I). Comme support ou excipient de la composition pharmaceutique selon l'invention, on peut utiliser tout

support ou excipient conventionnel non toxique.

Afin d'illustrer la demande et sans aucun caractère limitatif on va maintenant donner à titre d'illustration des exemples de préparation des composés de formule (I) ainsi que des exemples d'utilisation de ces composés dans les domaines cosmétiques et pharmaceutiques.

## EXEMPLE I

### Préparation du méthoxy-4 tétraméthyl-5,5,8,8 tétrahydro-5,6,7,8 naphtalénol-1

#### (a)Dichloro-2,5 diméthyl-2,5 hexane

Une suspension de 14,6g de diméthyl-2,5 hexanediol-2,5 dans 150cm³ d'acide chlorhydrique concentré est agitée, pendant une heure, à une température d'environ 5°C. Le solide est alors essoré, lavé abondamment à l'eau puis séché à température ambiante sur potasse et sous pression réduite. On obtient 16,5g de dichloro-2,5 diméthyl-2,5 hexane sous forme de cristaux blancs fondant à 65°C.

#### (b) Méthoxy-4 tétraméthyl-5,5,8,8 tétrahydro-5,6,7,8 naphtalénol-1

A une température comprise entre 0° et 5°C, sous atmosphère inerte, on ajoute 10,8g de chlorure d'aluminium anhydre à une suspension de 49,7g d'hydroxy-4 anisole et de 73,2g de dichloro-2,5 diméthyl-2,5 hexane, obtenu ci-dessus en (a) dans 230cm³ de dichloro-1,2 éthane anhydre.

Cinq minutes après l'introduction du chlorure d'aluminium, le milieu devient homogène et se colore progressivement en rouge-brun. Ce mélange est alors agité pendant cinq heures à température ambiante, temps au bout duquel la majorité du produit de départ est transformée. On ajoute alors 200cm³ d'eau glacée, le mélange est agité pendant 1/4 d'heure puis la phase organique est décantée. La phase aqueuse est extraite deux fois par 150cm³ de chlorure de méthylène. Les phases organiques, rassemblées, sont lavées à l'eau, séchées sur sulfate de sodium et le solvant est évaporé sous pression réduite. Le produit brut est alors recristallisé dans l'hexane. On obtient 50g de méthoxy-4 tétraméthyl-5,5,8,8 tétrahydro-5,6,7,8 naphtalénol-1 sous forme de cristaux blancs de point de fusion: 127°C.

Analyse élémentaire: $C_{15}H_{22}O_2$

| | | | |
|---|---|---|---|
| Calc : | C : 76,88 | H : 9,46 | O : 13,65 |
| Tr: | 76,87 | 9,50 | 13,73 |

## EXEMPLE II

### Préparation de l'acétyl-2 méthoxy-4 tétraméthyl-5,5,8,8 tétrahydro-5,6,7,8 naphtalénol-1

A un mélange, agité sous atmosphère inerte, à une température comprise entre 0° et 5°C, de 20,4g de méthoxy-4 tétraméthyl-5,5,8,8 tétrahydro-5,6,7,8 naphtalénol-1 obtenu à l'exemple I et de 6,18cm³ de chlorure d'acétyle dans 80cm³ de dichloro-1,2 éthane, on ajoute par petites portions 17,4g de chlorure d'aluminium anhydre en environ 1/2 heure de façon à ce que la température n'excède pas 5°C. A la fin de l'introduction le mélange est encore agité une heure à cette température puis une heure à température ambiante. La solution est alors versée dans 100cm³ d'eau glacée. On obtient à ce stade une émulsion qui est extraite trois fois par 100cm³ de chlorure de méthylène. Les phases organiques sont rassemblées, lavées à l'eau jusqu'à pH neutre des eaux de lavage, séchées sur sulfate de sodium et le solvant éliminé par évaporation sous vide.

Le produit brut obtenu est purifié par chromatographie sur gel de silice. Le dérivé acylé attendu est élué au mélange hexane-chlorure de méthylène 60-40. Après évaporation des phases éluantes puis recristallisation dans l'éthanol on obtient 12g d'acétyl-2 méthoxy-4 tétraméthyl-5,5,8,8 tétrahydro-5,6,7,8 naphtalénol-1 sous forme d'aiguilles jaune clair de point de fusion: 90°C.

Analyse élémentaire: $C_{17}H_{24}O_3$

| | | | |
|---|---|---|---|
| Calc: | C : 73,88 | H : 8,75 | O : 17,37 |
| Tr: | 73,87 | 8,84 | 17,20 |

Dans les dernières fractions de chromatographie un produit secondaire est entrainé correspondant à une O-acylation. Il s'agit de l'acétoxy-1 méthoxy-4 tétraméthyl-5,5,8,8 tétrahydro-5,6,7,8 naphtalène sous forme de cristaux blancs de point de fusion: 90°C.

EXEMPLE III

Préparation de l'(hydroxy-1 éthyl)-2 méthoxy-4 tétraméthyl-5,5,8,8 tétrahydro-5,6,7,8 naphtalénol-1

A une solution agitée, à température ambiante sous atmosphère inerte, de 2,9g d'acétyl-2 méthoxy-4 tétraméthyl-5,5,8,8 tétrahydro-5,6,7,8 naphtalénol-1 obtenu à l'exemple II, dans un mélange de 40cm³ de méthanol et 5cm³ de tétrahydrofuranne, on ajoute en environ 20mn par petites portions 1,5g de borohydrure de sodium. Après la fin de l'introduction la solution est encore agitée une heure à température ambiante puis refroidie vers 5°C, température à laquelle on introduit sous agitation 100cm³ d'eau. Le mélange est acidifié sous agitation par addition de 45cm³ d'acide chlorhydrique 1N puis extrait 3 fois par 50cm³ d'éther éthylique. Les phases éthérées sont rassemblées, lavées à l'eau, séchées sur sulfate de sodium puis concentrées à sec.

On obtient 3g d'un solide jaune que l'on purifie par passage sur colonne de gel de silice que l'on élue au chlorure de méthylène.

Après évaporation de l'éluant, on obtient 2,4g d'une poudre jaune clair de point de fusion: 99°C.

Analyse élémentaire: $C_{17}H_{26}O_3$

| | | | |
|---|---|---|---|
| Calc: | C : 73,35 | H : 9,41 | O : 17,24 |
| Tr: | 73,25 | 9,40 | 17,44 |

EXEMPLE IV

Préparation de l'(hydroxy-2 propyl-2)-2 méthoxy-4 tétraméthyl-5,5,8,8 tétrahydro-5,6,7,8 naphtalénol-1

A une solution agitée, à -25°C sous atmosphère inerte, de 7,3g d'acétyl-2 méthoxy-4 tétraméthyl-5,5,8,8 tétrahydro-5,6,7,8 naphtalénol-1, obtenu à l'exemple II, dans 120cm³ d'éther éthylique anhydre, on ajoute en environ 1/4 d'heure 19cm³ de solution 3M de bromure de méthylmagnésium dans l'éther.

On observe la formation d'un précipité jaune et la température à la fin de l'introduction du magnésien est de -15°C. L'agitation est maintenue 1/2 heure supplémentaire à cette température, puis une heure à 10°C. La majorité du produit de départ est alors transformée. Le mélange est hydrolysé à 0°C par addition de 25cm³ d'eau puis de 60cm³ d'acide chlorhydrique 1N. La phase éthérée est décantée et la phase aqueuse est extraite 2 fois par 100cm³ d'éther éthylique.

Les phases organiques rassemblées sont lavées à l'eau, séchées sur sulfate de sodium et concentrées. Le solide blanc obtenu est recristallisé dans l'hexane en présence d'une trace d'acétone. On obtient 7g d'(hydroxy-2 propyl-2)-2 méthoxy-4 tétraméthyl-5,5,8,8 tétrahydro-5,6,7,8 naphtalénol-1 sous forme de cristaux blancs de point de fusion: 140°C.

Analyse élémentaire: $C_{18}H_{28}O_3$

| | | | |
|---|---|---|---|
| Calc : | C : 73,93 | H : 9,65 | O : 16,41 |
| Tr: | 74,02 | 9,62 | 16,30 |

EXEMPLE V

Préparation du (propényl-2)-2 méthoxy-4 tétraméthyl-5,5,8,8 tétrahydro-5,6,7,8 naphtalénol-1

A température ambiante, on ajoute goutte à goutte $30cm^3$ d'une solution d'acide sulfurique à 50% à 2g d'(hydroxy-2 propyl-2)-2 méthoxy-4 tétraméthyl-5,5,8,8 tétrahydro-5,6,7,8 naphtalénol-1, obtenu à l'exemple IV, solubilisé dans $30cm^3$ de chlorure de méthylène. Le mélange est abandonné une nuit à température ambiante. La phase de chlorure de méthylène est décantée, lavée à l'eau jusqu'à pH neutre des eaux de lavage, séchée sur sulfate de sodium puis concentrée. Le produit attendu est purifié par passage sur colonne de chromatographie de gel de silice. Il est élué au mélange chlorure de méthylène-hexane (50:50).

Après évaporation de l'éluant, on obtient 1,3g d'un liquide jaune clair dont le spectre $^1H$ RMN 80MHz correspond à la structure attendue.

EXEMPLE VI

Préparation de l'acide hydroxy-1 méthoxy-4 tétraméthyl-5,5,8,8 tétrahydro-5,6,7,8 naphtalène carboxylique-2

(a) Diméthoxy-1,4 tétraméthyl-5,5,8,8 tétrahydro-5,6,7,8 naphtalène

A une solution, agitée à 0°C sous atmosphère inerte, de 165,5g de diméthoxy-1,4 benzène et de 218,7g de dichloro-2,5 diméthyl-2,5 hexane, obtenu à l'exemple 1(a), dans $750cm^3$ de dichloro-1,2 éthane anhydre, on ajoute en une fois 31,9g de chlorure d'aluminium anhydre en poudre. Ensuite le mélange est agité pendant une heure à température ambiante puis abandonné une nuit. Le lendemain après 4 heures d'agitation à cette température, $600cm^3$ d'eau glacée sont ajoutés. La phase organique est décantée et la phase aqueuse est extraite trois fois par $400cm^3$ de chlorure de méthylène. Les phases organiques sont rassemblées, lavées à l'eau jusqu'à pH neutre des eaux de lavage puis séchées sur sulfate de sodium et concentrées. Le produit brut obtenu est traité au noir animal dans l'éthanol bouillant puis recristallisé dans ce solvant. On obtient 195g de diméthoxy-1,4 tétraméthyl-5,5,8,8 tétrahydro-5,6,7,8 naphtalène sous forme d'aiguilles blanches de point de fusion: 195°C.

(b) Acétyl-2 diméthoxy-1,4 tétraméthyl-5,5,8,8 tétrahydro 5,6,7,8 naphtalène

A une solution, agitée à une température d'environ -10°C, de 74,4g de diméthoxy-1,4 tétraméthyl-5,5,8,8 tétrahydro-5,6,7,8 naphtalène obtenu ci-dessus en (a) et de $21,3cm^3$ de chlorure d'acétyle dans $380cm^3$ de dichloro-1,2 éthane on ajoute par petites quantités 48g de chlorure d'aluminium anhydre en environ 3/4 d'heure. Après la fin de l'addition, le mélange est agité 1h30mn à 0°C, dilué par addition de $700cm^3$ d'eau glacée, puis la phase organique est décantée, lavée plusieurs fois à l'eau jusqu'à pH neutre des eaux de lavage et séchée sur sulfate de sodium. Après élimination du solvant par distillation sous vide, le produit brut obtenu est recristallisé dans l'éthanol. On obtient 76g d'acétyl-2 diméthoxy-1,4 tétraméthyl-5,5,8,8 tétrahydro-5,6,7,8 naphtalène sous forme d'aiguilles blanches de point de fusion: 97°C.

Analyse élémentaire : $C_{18}H_{26}O_3$

| | | | |
|---|---|---|---|
| Calc: | C : 74,45 | H : 9,02 | O : 16,53 |
| Tr: | 74,42 | 8,99 | 16,62 |

(c) Acide diméthoxy-1,4 tétraméthyl-5,5,8,8 tétrahydro-5,6,7,8 naphtalène carboxylique-2

On prépare dans un premier stade une solution d'hypobromite de sodium en ajoutant au goutte-à-goutte $77cm^3$ de brome à une solution contenant 192,7g de soude dans $890cm^3$ d'eau refroidie entre 0° et 5°C. Le mélange est gardé sous agitation à 0°C.

Dans un deuxième stade, on ajoute goutte à goutte cette solution d'hypobromite à une solution agitée refroidie vers 5°C de 43g d'acétyl-2 diméthoxy-1,4 tétraméthyl-5,5,8,8 tétrahydro-5,6,7,8 naphtalène, obtenu ci-dessus en (b) dans $500cm^3$ de dioxanne. La vitesse d'introduction est telle que la température ne dépasse pas 15°C. Le mélange réactionnel est ensuite abandonné une nuit, dilué à l'aide de $300cm^3$ d'eau, puis, à une température inférieure à 20°C, l'excès d'hypobromite de sodium est détruit par addition lente d'une solution aqueuse à 25% de bisulfite de sodium. L'acide attendu, est ensuite précipité par acidification du mélange en ajoutant 380cm³ d'acide chlorhydrique 6N. Le précipité est essoré, lavé à l'eau et séché sous pression réduite. On obtient 40,8g d'acide diméthoxy-I,4 tétraméthyl-5,5,8,8 tétrahydro-5,6,7,8 naphtalène carboxylique-2 sous forme d'un solide blanc de point de fusion: 187°C.

**Analyse élémentaire: $C_{17}H_{24}O_4$**

| | | | |
|---|---|---|---|
| **Calc:** | **C : 69,84** | **H : 8,27** | **O : 21,89** |
| **Tr:** | **69,56** | **8,29** | **21,64** |

(d) Acide hydroxy-1 méthoxy-4 tétraméthyl-5,5,8,8 tétrahydro-5,6,7,8 naphtalène carboxylique-2

A une solution agitée, à la température ordinaire, sous atmosphère inerte, de 30g d'acide diméthoxy-1,4 tétraméthyl-5,5,8,8 tétrzhydro-5,6,7,8 naphtalène carboxylique-2 obtenu ci-dessus en (c) dans 150cm³ d'acide acétique on ajoute rapidement 300cm³ d'une solution à 57Z d'acide iodhydrique. Le mélange est ensuite porté sous reflux pendant 1/2 heure puis la température est ramenée vers 30°C, température à laquelle il est versé dans 1250cm³ d'eau glacée. Le précipité formé est essoré, lavé plusieurs fois à l'eau jusqu'à pH neutre des eaux de lavage puis séché.

On obtient 27g d'acide hydroxy-1 méthoxy-4 tétraméthyl-5,5,8,8 tétrahydro-5,6,7,8 naphtalène carboxylique-2 contenant une petite quantité d'acide dihydroxy-1,4 tétraméthyl-5,5,8,8 tétrahydro-5,6,7,8 naphtalène carboxylique-2.

Ce mélange est fractionné par chromatographie sur colonne de gel de silice. Le produit principal est élué au mélange acide acétique-dioxanne- toluène 2.8.90. Après évaporation de l'éluant et recristallisation dans le toluène on obtient 10g d'acide hydroxy-1 méthoxy-4 tétraméthyl-5,5,8,8 tétrahydro-5,6,7,8 naphtalène carboxylique-2 sous forme de cristaux blancs de point de fusion: 213°C.

Analyse élémentaire: $C_{16}H_{22}O_4$

| | | | |
|---|---|---|---|
| Calc: | C : 69,04 | H : 7,97 | O : 22,99 |
| Tr: | 69,14 | 8,01 | 23,08. |

Dans les fractions d'élution suivantes, l'acide dihydroxy-1,4 tétraméthyl-5,5,8,8 tétrahydro-5,6,7,8 naphtalène carboxylique-2 est entrainé. Après évaporation de l'éluant, on obtient 3g de cristaux beiges de point de fusion: 215°C.

Analyse élémentaire : $C_{15}H_{20}O_4$

| | | | |
|---|---|---|---|
| Calc: | C : 68,16 | H : 7,63 | O : 24,21 |
| Tr: | 68,20 | 7,61 | 24,32 |

Si l'on souhaite obtenir ce dernier acide comme produit principal on maintient alors le mélange réactionnel au moins 3 heures sous reflux.

EXEMPLE VII

Préparation du dihydroxy-1,4 tétraméthyl-5,5,8,8 tétrahydro-5,6,7,8 naphtalène

a) Tétraméthyl-5,5,8,8 tétrahydro-5,6,7,8 naphtoquinone-1,4 :

A une solution de 17,42g (0,07 mole) de diméthoxy-1,4 tétraméthyl-5,5,8,8 tétrahydro-5,6,7,8 naphtalène obtenu à l'exemple VIa dans 340 cm³ de dioxanne, refroidie à + 5°C, on ajoute sous agitation 34,7g (0,28 mole) d'oxyde d'argent AgO(II) puis en goutte-à-goutte rapide 70 cm³ (0,42 mole) d'acide nitrique 6N.

On supprime le refroidissement et agite jusqu'à consommation totale de l'oxyde d'argent (10 à 15 mn) puis ajoute 500cm³ de dichlorométhane et 200 cm³ d'eau.

On sépare la phase organique par décantation, lave par 200cm³ d'eau, sèche sur sulfate de sodium et évapore à sec. L'huile rouge obtenue est chromatographiée sur gel de silice dans le mélange éluant hexane/dichlorométhane 60/40.

Après évaporation et séchage, on obtient 2,7g de tétraméthyl-5,5,8,8 tétrahydro-5,6,7,8 naphtoquinone-1,4 sous la forme d'une huile rouge.

Le spectre RMN $^1H$ 80 MHz est conforme à la structure attendue.

Analyse élémentaire : $C_{14}H_{18}O_2$

| | | | |
|---|---|---|---|
| Calc : | C : 77,03 | H : 8,31 | O : 14,66 |
| Tr : | 76,87 | 8,28 | 14,68 |

b) Dihydroxy-1,4 tétraméthyl-5,5,8,8 tétrahydro-5,6,7,8 naphtalène

A une suspension de 40mg (~ 1 mmole) d'hydrure de lithium aluminium dans 2 $cm^3$ d'éther éthylique anhydre, agitée sous atmosphère inerte et refroidie à + 5°C, on ajoute goutte à goutte une solution de 0,22g (~ 1 mmole) de tétraméthyl-5,5,8,8 tétrahydro-5,6,7,8 naphtoquinone-1,4 obtenue ci-dessus en (a) dans 2 $cm^3$ d'éther éthylique anhydre. Après 30 mn d'agitation on ajoute goutte à goutte de l'eau pour détruire l'excès d'hydrure, puis 30 $cm^3$ d'éther éthylique et enfin quelques gouttes d'acide chlorhydrique N jusqu'à pH acide.

La phase éthérée est séparée, lavée à l'eau, séchée sur sulfate de sodium et évaporée à sec sous atmosphère inerte.

On obtient 0,2g de dihydroxy-1,4 tétraméthyl-5,5,8,8 tétrahydro-5,6,7,8 naphtalène sous la forme d'un solide beige de point de fusion : 114°C.

Le spectre RMN $^1HCDCl_3$ 80 MHz sous atmosphère inerte est conforme à la structure attendue ($\delta$ =1,25 ppm,s,12H,4$CH_3$ ; $\delta$ =1,64ppm,s,4H,2$CH_2$ ; $\delta$ = 6,90 ppm, 2H aromatiques).

Lorsque la solution dans le deutérochloroforme n'est pas conservée sous gaz inerte on observe la présence de signaux parasites correspondant à la quinone ($\delta$ = 1,29 ppm,s, $CH_3$; $\delta$ = 1,51 ppm,s, $CH_2$ ; H quinoniques). Après 5 heures en solution on n'observe plus que la quinone.

Analyse élémentaire : $C_{14}H_{20}O_2$

| | | | |
|---|---|---|---|
| Calc : | C : 76,32 | H : 9,15 | O : 14,52 |
| Tr : | 76,37 | 8,98 | 14,60 |

EXEMPLE VIII

Préparation du benzoyl-2 méthoxy-4-tétraméthyl-5,5,8,8 tétrahydro-5,6,7,8 naphtalénol-1 :

A une solution refroidie à + 5°C, de 23,44g (0,1 mole) de méthoxy-4 tétraméthyl-5,5,8,8 tétrahydro-5,6,7,8 naphtalénol-1 obtenu à l'exemple I, dans 400 $cm^3$ de dichloro-1,2 éthane anhydre et 11,7 $cm^3$ (14,06g-0,1 mole) de chlorure de benzoyle, on ajoute par portions 16g (0,12 mole) de chlorure d'aluminium anhydre en environ 30 mn.

A la fin de l'introduction, le mélange est agité 2h en laissant revenir à la température ambiante.

La solution est alors versée dans 250 $cm^3$ d'eau glacée. La phase aqueuse est séparée par décantation et réextraite 3 fois par 200 $cm^3$ de dichlorométhane. Les phases organiques sont réunies, lavées à l'eau jusqu'à pH neutre des eaux de lavage, séchées sur sulfate de sodium et évaporées à sec sous pression réduite.

Le produit brut obtenu (32,2g) est purifié par chromatographie sur gel de silice avec le mélange éluant heptane/dichlorométhane 90/10.

Après évaporation des phases éluantes puis séchage sous vide à 60°C, on obtient 19,95g de benzoyl-2 méthoxy-4 tétraméthyl-5,5,8,8 tétrahydro-5,6,7,8 naphtalénol-1 sous la forme d'un solide jaune de point de fusion : 85°C.

Le spectre RMN $^1H$ 80 MHz est conforme à la structure attendue.

EXEMPLE IX

Préparation du benzyl-2 méthoxy-4 tétraméthyl-5,5,8,8 tétrahydro-5,6,7,8 naphtalénol-1:

A une suspension de 30 mmoles d'hydrure de sodium dans 5 $cm^3$ de toluène, agitée à température ambiante sous atmosphère inerte, on ajoute lentement une solution de 7,22g (30 mmoles) de méthoxy-4 tétraméthyl-5,5,8,8 tétrahydro-5,6,7,8 naphtalénol-1 obtenu à l'exemple I, dans environ 20 $cm^3$ de toluène en maintenant la température à 20°C. La solution obtenue est chauffée au reflux puis on coule goutte à goutte 4,33g (33,9 mmoles) de chlorure de benzyle en environ 30 mn. Le reflux est maintenu 5 heures. Après refroidissement à température ambiante on ajoute lentement 40 $cm^3$ d'acide chlorhydrique N, agite 15 mn et sépare les 2 phases par décantation. La phase aqueuse est réextraite deux fois par 200 $cm^3$ de toluène. Les phases organiques sont rassemblées, lavées à l'eau, séchées sur sulfate de sodium et concentrées sous pression réduite. L'huile obtenue est purifiée par chromatographie sur gel de silice dans le mélange éluant heptane / dichlorométhane 90/10 suivie d'une recristallisation dans l'alcool éthylique à 96°C.

Après essorage et séchage sous vide à 60°C on obtient 2,2g de benzyl-2 méthoxy-4 tétraméthyl-5,5,8,8 tétrahydro-5,6,7,8 naphtalénol-1 sous la forme d'un solide blanc de point de fusion : 80°C.

Le spectre RMN $^1H$ 250 MHz est conforme à la structure attendue.

Analyse élémentaire : $C_{22}H_{28}O_2$

| | | | |
|---|---|---|---|
| Calc : | C : 81,44 | H : 8,70 | O : 9,86 |
| Tr : | 81,48 | 8,63 | 10,03 |

EXEMPLES DE COMPOSITIONS

EXEMPLE A

GEL

| | |
|---|---|
| (hydroxy-1 éthyl)-2 méthoxy-4 tétraméthyl-5,5,8,8 tétrahydro-5,6,7,8 naphtalénol-1..... | 0,12 |
| Polymère carboxyvinylique vendu par la Société GOODRICH sous la dénomination de "Carbopol 934"............................... | 0,80 |
| Glycérine................................... | 12,00 |
| Ethanol...................................... | 15,00 |
| Conservateur................................ | 0,20 |
| Parfum....................................... | 0,20 |
| Triéthanolamine qs ..................pH 5,3 | |
| Eau déminéralisée......qsp.................. | 100,00g |

EXEMPLE B

HUILE

| | |
|---|---|
| Méthoxy-4 tétraméthyl-5,5,8,8 tétrahydro-5,6,7,8 naphtalénol-1 | 0,60 |
| Benzoate d'alcools en $C_{12}$-$C_{15}$ vendu par la Société FINETEX sous la dénomination "FINSOLV TN" | 30,00 |
| Huile de tournesol raffinée stabilisée | 20,00 |
| Parfum | 1,00 |
| Huile de silicone vendue par la Société UNION CARBIDE sous la dénomination de "Volatile silicone 7207" qsp | 100,00g |

EXEMPLE C

LAIT SOUS FORME D'UNE EMULSION EAU-DANS-L'HUILE

| | |
|---|---|
| (hydroxy-2 propyl-2)-2 méthoxy-4 tétraméthyl-5,5,8,8 tétrahydro-5,6,7,8 naphtalénol-1 | 0,25 |
| Benzylidène camphre | 2,00 |
| Mélange d'esters d'acides gras, d'esters polyglycérolés et de tensio-actifs siliconés vendu par la Société GOLDSCHMIDT sous la dénomination "ABIL WS08" | 5,00 |
| Vaseline blanche | 2,00 |
| Cire d'abeilles | 2,50 |
| Benzoate d'alcools en $C_{12}$-$C_{15}$ vendu par la Société FINETEX sous la dénomination "FINSOLV TN" | 19,00 |
| Glycérine | 5,00 |
| Chlorure de sodium | 2,00 |
| Parfum | 0,40 |
| Conservateur | 0,20 |
| Eau déminéralisée qsp | 100,00g |

EXEMPLE D

LAIT

| | |
|---|---|
| (hydroxy-1 éthyl)-2 méthoxy-4 tétraméthyl-5,5,8,8 tétrahydro-5,6,7,8 naphtalénol-1..... | 1,50 |
| p-hydroxycinnamate de 2-éthyl hexyle (filtre solaire)............................ | 3,50 |
| 2-hydroxy-4-méthoxy benzophénone (filtre solaire).................................... | 1,00 |
| Alcool cétylique............................ | 1,00 |
| Alcool oléocétylique à 30 moles OE vendu par la Société HENKEL sous la dénomination de "MERGITAL OC30"........................... | 5,00 |
| Alcool stéarylique.......................... | 4,00 |
| Hexadécanoyloxy-1 (éthyl-2' hexyléther)-3 propanol-2................................ | 2,00 |
| Mélange 90/10 d'éthyl-2 hexanoate de cétostéaryle et de myristate d'isopropyle vendu par la Société CREATIONS AROMATIQUES sous la dénomination "CERAMOLL"........................... | 2,00 |
| Huile de vaseline........................... | 8,00 |
| Propylène glycol............................ | 4,00 |
| Conservateur................................ | 0,20 |
| Parfum...................................... | 0,40 |
| Eau déminéralisée..........qsp............... | 100,00g |

EXEMPLE E

STICK

| | |
|---|---|
| Méthoxy-4 tétraméthyl-5,5,8,8 tétrahydro-5,6,7,8 naphtalénol-1 | 1,00 |
| Ozokérite | 20,00 |
| Cire d'abeilles | 7,00 |
| Alcool oléique | 12,00 |
| Lanoline hydrogénée | 8,00 |
| Huile de lanoline | 8,00 |
| Cire de carnauba | 1,00 |
| Benzoate d'alcools en $C_{12}$-$C_{15}$ vendu par la Société FINETEX sous la dénomination "FINSOLV TN" | 17,00 |
| Octaméthylcyclotetrasiloxane vendu par la Société GOLDSCHMIDT sous la dénomination "ABIL K4" | 3,00 |
| Huile de vaseline qsp | 100,00g |

EXEMPLE F

CREME SOUS FORME D'UNE EMULSION HUILE-DANS-L'EAU

| | |
|---|---|
| (hydroxy-1 éthyl)-2 méthoxy-4 tétraméthyl-5,5,8,8 tétrahydro-5,6,7,8 naphtalénol-1 | 0,50 |
| Méthyl sulfate de (oxo-2 bornylidène-3) méthyl -4 phényl triméthylammonium | 4,00 |
| Lactate de sodium | 1,00 |
| Alcool céto-stéarylique et alcools gras oxyéthylénés vendu par la Société SINNOVA sous la dénomination "SINNOWAX AO" | 7,50 |
| Mélange de mono et distéarate de glycérol non auto-émulsionnable | 2,10 |
| Alcool cétylique | 1,00 |
| Alcool myristique | 0,60 |
| Sorbitol à 70% | 3,00 |
| Palmitate d'isopropyle | 10,00 |
| Huile de vaseline | 7,00 |
| Conservateur | 0,20 |
| Parfum | 0,60 |
| Eau déminéralisée qsp | 100,00g |

Compositions pharmaceutiques par voie topique

EXEMPLE G

ONGUENT

| | |
|---|---|
| Méthoxy-4 tétraméthyl-5,5,8,8 tétrahydro-5,6,7,8 naphtalénol-1 | 2,00 |
| Huile de vaseline fluide | 9,10 |
| Silice vendue par la Société DEGUSSA sous la dénomination de "AEROSIL 200" | 9,20 |
| Myristate d'isopropyle......qsp | 100g |

Dans cet exemple le composé actif peut être remplacé par la même quantité de benzyl-2-méthoxy-4 tétraméthyl-5,5,8,8 tétrahydro-5,6,7,8 naphtalénol-1.

EXEMPLE H

CREME HUILE DANS L'EAU ANIONIQUE

| | |
|---|---|
| (hydroxy-1 éthyl)-2 méthoxy-4 tétraméthyl-5,5,8,8 tétrahydro-5,6,7,8 naphtalénol-1 | 3,00 |
| Dodécyl sulfate de sodium | 0,80 |
| Glycérol | 2,00 |
| Alcool stéarylique | 20,0 |
| Triglycérides d'acides caprique/caprylique vendus par la Société DYNAMIT NOBEL sous la dénomination de "MIGLYOL 812" | 20,0 |
| Conservateurs | qs |
| Eau déminéralisée.........qsp | 100g |

Dans cet exemple le composé actif peut être remplacé par la même quantité de benzyl-2 méthoxy-4 tétraméthyl-5,5,8,8 tétrahydro-5,6,7,8 naphtalénol-1.

EXEMPLE I

GEL

| | |
|---|---|
| (hydroxy-2 propyl-2)-2 méthoxy-4 tétraméthyl-5,5,8,8 tétrahydro-5,6,7,8 naphtalénol-1 | 1,00 |
| Hydroxypropyl cellulose vendue par la Société HERCULES sous la dénomination de "KLUCEL HF" | 2,00 |
| Ethanol | 70,0 |

Eau....................qsp................. 100g

Dans cet exemple le composé actif peut être remplacé par 0,5g de benzyl-2 méthoxy-4 tétraméthyl-5,5,8,8 tétrahydro-5,6,7,8 naphtalénol-1.

## Revendications

1. Dérivés de tétrahydro-5,6,7,8 naphtalénol-1 répondant à la formule générale suivante:

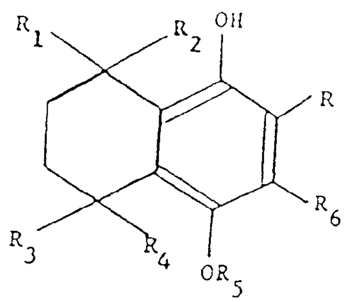

(I)

dans laquelle:

$R_1$, $R_2$, $R_3$ et $R_4$ représentent un radical alkyle inférieur,

$R_5$ et $R_6$ représentent un atome d'hydrogène ou un radical alkyle inférieur,

R représente un atome d'hydrogène, un radical alkyle en $C_1$-$C_{18}$, un radical alkyle en $C_2$-$C_{18}$ substitué par un ou plusieurs hydroxyle(s), un radical alkényle en $C_3$-$C_{18}$, un radical acyle en $C_2$-$C_{18}$, un radical benzyle, un radical benzoyle, un radical carboxyle et les sels carboxyliques d'un métal alcalin ou alcalino-terreux ou d'une amine organique.

2. Composés selon la revendication 1, caractérisés par le fait que les radicaux alkyles inférieurs et ceux ayant jusqu'à 18 atomes de carbone sont pris dans le groupe constitué par les radicaux méthyle, éthyle, isopropyle, isobutyle, butyle, tertiobutyle, éthyl-2 hexyle, isooctyle, dodécyle, hexadécyle et octadécyle.

3. Composés selon la revendication 1, caractérisés par le fait que les radicaux alkyles en $C_2$- $C_{18}$ substitués par un ou plusieurs hydroxyle(s) sont pris dans le groupe constitué par les radicaux hydroxy-1 éthyle, hydroxy-2 propyle, hydroxy-1 propyle, hydroxy-1 butyle, hydroxy-1 hexyle, hydroxy-1 éthyl-1 hexyle ou dihydroxy-2,3 propyle.

4. Composés selon la revendication 1, caractérisés par le fait que les radicaux alkényles sont pris dans le groupe constitué par les radicaux propényle, butényle, hexényle, octényle, dodécényle et octadécényle.

5. Composés selon la revendication 1, caractérisés par le fait que les radicaux acyles sont pris dans le groupe constitué par les radicaux acétyle, propionyle, butyryle, isobutyryle, hexanoyle, octanoyle, dodécanoyle et octadécanoyle.

6. Composés selon la revendication 1, caractérisés par le fait que lorsque R est un radical benzyle ou benzoyle, celui-ci peut être représenté par la formule générale suivante:

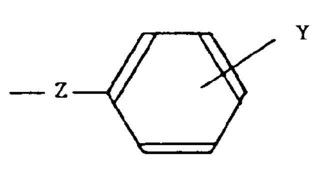

dans laquelle:

Z représente -CO-, -CHOH- ou $-CH_2$ - et

Y représente un atome d'hydrogène, un halogène de préférence le chlore, un alcoxy de préférence méthoxy ou le radical trifluorométhyle.

**7.** Composés selon la revendication 1, caractérisés par le fait qu'ils se présentent sous forme d'un sel de sodium, potassium, magnésium ou de triéthanolamine lorsque R =-COOH.

**8.** Composés selon l'une quelconque des revendications 1 à 7, caractérisés par le fait qu'ils sont pris dans le groupe constitué par:

- Méthoxy-4 tétraméthyl-5,5,8,8 tétrahydro-5,6,7,8 naphtalénol-1,
- Acétyl-2 méthoxy-4 tétraméthyl-5,5,8,8 tétrahydro-5,6,7,8 naphtalénol-1,
- (hydroxy-1 éthyl)-2 méthoxy-4 tétraméthyl-5,5,8,8 tétrahydro-5,6,7,8 naphtalénol-1,
- (hydroxy-2 propyl-2)-2 méthoxy-4 tétraméthyl-5,5,8,8 tétrahydro-5,6,7,8 naphtalénol-1,
- (propényl-2)-2 méthoxy-4 tétraméthyl-5,5,8,8 tétrahydro-5,6,7,8 naphtalénol-1,
- Acide hydroxy-1 méthoxy-4 tétraméthyl-5,5,8,8 tétrahydro-5,6,7,8 naphtalène carboxylique,
- Dihydroxy-1,4 tétraméthyl-5,5,8,8 tétrahydro-5,6,7,8 naphtalène,
- Benzoyl-2 méthoxy-4 tétraméthyl-5,5,8,8 tétrahydro-5,6,7,8 naphtalénol-1 et
- Benzyl-2 méthoxy-4 tétraméthyl-5,5,8,8 tétrahydro-5,6,7,8 naphtalénol-1.

**9.** Procédé de préparation des composés selon les revendications 1 à 8 caractérisé par le fait qu'il consiste à faire réagir, dans un solvant organique en présence d'un acide de Lewis, un dichloro-2,5 alcane de formule:

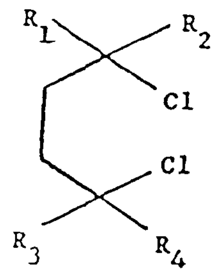

dans laquelle:

$R_1$ , $R_2$ , $R_3$ et $R_4$ représentent un radical alkyle inférieur, sur un alcoxy-4 phénol ou un dialcoxy-1,4 benzène de formule:

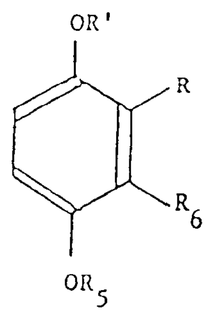

dans laquelle:

R , et $R_5$ et $R_5$ ont les mêmes significations que celles données à la revendication 1, et R' représente un atome d'hydrogène ou un radical alkyle inférieur, et que l'on soumet le dérivé dialcoxy-1,4 obtenu (lorsque R' représente un radical alkyle) à un traitement en présence d'acide iodhydrique ou bromhydrique.

**10.** Procédé selon la revendication 9, caractérisé par le fait que le solvant organique est le dichloro-1,2 éthane et l'acide de Lewis est le chlorure d'aluminium.

11. Composition cosmétique, caractérisée par le fait qu'elle contient une quantité efficace d'au moins un composé de formule (I) selon l'une quelconque des revendications 1 à 8 dans un support cosmétiquement acceptable contenant au moins une phase grasse.

12. Composition selon la revendication 11, caractérisée par le fait qu'elle contient au moins un composé choisi dans le groupe constitué par:
- Méthoxy-4 tétraméthyl-5,5,8,8 tétrahydro-5,6,7,8 naphtalénol-1,
- Acétyl-2 méthoxy-4 tétraméthyl-5,5,8,8 tétrahydro-5,6,7,8 naphtalénol-1,
- (hydroxy-1 éthyl)-2 méthoxy-4 tétraméthyl-5,5,8,8 tétrahydro-5,6,7,8 naphtalénol-1,
- (hydroxy-2 propyl-2)-2 méthoxy-4 tétraméthyl-5,5,8,8 tétrahydro-5,6,7,8 naphtalénol-1,
- (propényl-2)-2 méthoxy-4 tétraméthyl-5,5,8,8 tétrahydro-5,6,7,8 naphtalénol-1,
- Acide hydroxy-1 méthoxy-4 tétraméthyl-5,5,8,8 tétrahydro-5,6,7,8 naphtalène carboxylique,
- Dihydroxy-1,4 tétraméthyl-5,5,8,8 tétrahydro-5,6,7,8 naphtalène,
- Benzoyl-2 méthoxy-4 tétraméthyl-5,5,8,8 tétrahydro-5,6,7,8 naphtalénol-1 et
- Benzyl-2 méthoxy-4 tétraméthyl-5,5,8,8 tétrahydro-5,6,7,8 naphtalénol-1.

13. Composition selon l'une quelconque des revendications 11 et 12, caractérisée par le fait qu'elle contient le composé de formule (I) en une proportion comprise entre 0,05 et 10% en poids par rapport au poids total de la composition.

14. Procédé de protection d'une composition cosmétique contre l'oxydation, caractérisé par le fait qu'il consiste à incorporer à cette composition une quantité efficace d'au moins un composé de formule (I).

15. Composition pharmaceutique, caractérisée par le fait qu'elle contient une quantité thérapeutiquement active d'au moins un composé de formule (I) selon les revendications 1 à 8 dans un support ou excipient pharmaceutique.

16. Composition pharmaceutique selon la revendication 15, caractérisée par le fait qu'elle contient le composé actif de formule (I) en une proportion comprise entre 0,01 et 20% et de préférence entre 0,1 et 5% en poids par rapport au poids total de la composition.

17. Composition pharmaceutique selon l'une des revendications 15 et 16, caractérisée par le fait que le composé actif de formule (I) est choisi dans le groupe constitué par:
- Méthoxy-4 tétraméthyl-5,5,8,8 tétrahydro-5,6,7,8 naphtalénol-1,
- Acétyl-2 méthoxy-4 tétraméthyl-5,5,8,8 tétrahydro-5,6,7,8 naphtalénol-1,
- (hydroxy-1 éthyl)-2 méthoxy-4 tétraméthyl-5,5,8,8 tétrahydro- 5,6,7,8 naphtalénol-1,
- (hydroxy-2 propyl-2)-2 méthoxy-4 tétraméthyl-5,5,8,8 tétrahydro-5,6,7,8 naphtalénol-1,
- (propényl-2)-2 méthoxy-4 tétraméthyl-5,5,8,8 tétrahydro-5,6,7,8 naphtalénol-1,
- Acide hydroxy-1 méthoxy-4 tétraméthyl-5,5,8,8 tétrahydro-5,6,7,8 naphtalène carboxylique,
- Dihydroxy-1,4 tétraméthyl-5,5,8,8 tétrahydro-5,6,7,8 naphtalène,
- Benzoyl-2 méthoxy-4 tétraméthyl-5,5,8,8 tétrahydro-5,6,7,8 naphtalénol-1 et
- Benzyl-2 méthoxy-4 tétraméthyl-5,5,8,8 tétrahydro-5,6,7,8 naphtalénol-1.

18. Utilisation d'un composé de formule (I) selon les revendications 1 à 8 pour la préparation d'une composition pharmaceutique destinée au traitement préventif des inflammations et allergies cutanées.

19. Utilisation d'un composé de formule (I) selon les revendications 1 à 8 pour la préparation d'une composition pharmaceutique destinée à la prévention de certains cancers.

**Patentansprüche**

1. Derivate von 5,6,7,8-Tetrahydro-l-naphthol der allgemeinen Formel

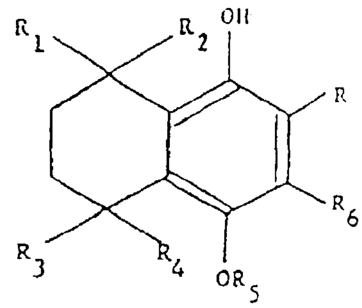

(I)

in der:
$R_1$, $R_2$, $R_3$ und $R_4$ einen Niedrigalkylrest,
$R_5$ und $R_6$ ein Wasserstoffatom oder einen Niedrigalkylrest,
R ein Wasserstoffatom, einen $C_1$-$C_{18}$-Alkylrest, einen durch eine oder mehrere Hydroxylgruppen substituierten $C_2$-$C_{18}$-Alkylrest, einen $C_3$-$C_{18}$-Alkenylrest, einen $C_2$-$C_{18}$-Acylrest, einen Benzylrest, einen Benzoylrest, einen Carboxylrest, oder die Carbonsäuresalze eines Alkali- oder Erdalkalimetalles oder eines organischen Amins bedeuten.

2. Verbindungen nach Anspruch 1, dadurch gekennzeichnet, daß die Niedrigalkylreste und die mit bis zu 18 Kohlenstoffatomen aus den Methyl-, Ethyl-, Isopropyl-, Isobutyl-, Butyl-, tert.-Butyl-, 2-Ethylhexyl-, Isooctyl-, Dodecyl-, Hexadecyl- und Octadecyl-Gruppen ausgewählt sind.

3. Verbindungen nach Anspruch 1, dadurch gekennzeichnet, daß die $C_2$-$C_{18}$-Alkylgruppen, die durch eine oder mehrere Hydroxylgruppen substituiert sein können, aus den 1-Hydroxyethyl-, 2-Hydroxypropyl-, 1-Hydroxypropyl-, 1-Hydroxybutyl-, 1-Hydroxyhexyl-, 1-Hydroxy 1-Ethylhexyl oder 2,3-Dihydroxypropyl-Gruppen ausgewählt sind.

4. Verbindung nach Anspruch 1, dadurch gekennzeichnet, daß die Alkenylgruppen aus den Propenyl-, Butenyl-, Hexenyl-, Octenyl-, Dodecenyl- und Octadecenyl-Gruppen ausgewählt sind.

5. Verbindungen nach Anspruch 1, dadurch gekennzeichnet, daß die Acylreste aus den Acetyl-, Propionyl-, Butyryl-, Isobutyryl-, Hexanoyl-, Octanoyl-, Dodecanoyl- und Octadecanoyl-Gruppen ausgewählt sind.

6. Verbindungen nach Anspruch 1, dadurch gekennzeichnet, daß R ein Benzyl- oder Benzoylrest der allgemeinen Formel ist:

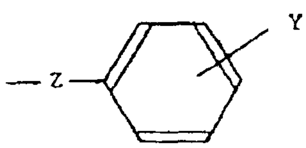

in der,
Z -CO-, -CHOH- oder $-CH_2-$ und
Y ein Wasserstoffatom, ein Halogenatom, vorzugsweise Chlor, einen Alkoxy-, vorzugsweise Methoxy oder den Trifluormethylrest bedeuten.

7. Verbindungen nach Anspruch 1, dadurch gekennzeichnet, daß sie als Natrium-, Kalium-, Magnesium- oder Triethanolaminsalz vorliegen, wenn R = -COOH bedeutet.

8. Verbindungen nach einem der Anspruche 1 bis 7, dadurch gekennzeichnet, daß sie aus folgender Gruppe ausgewählt sind:
- 4-Methoxy-5,5,8,8-tetramethy-5,6,7,8-tetrahydro-1-naphthol,
- 2-Acetyl-4-methoxy-5,5,8,8-tetramethyl-5,6,7,8-tetrahydro-1-naphthol,
- 2-(1-Hydroxyethyl)-4-methoxy-5,5,8,8-tetramethyl-5,6,7,8-tetrahydro-1-naphthol,
- 2-(2-Hydroxy-2-propyl)-4-methoxy-5,5,8,8-tetramethyl-5,6.7,8-tetrahydro-1-naphthol,
- 2-(2-Propenyl)-4-methoxy-5,5,8,8-tetramethyl-5,6,7,8-tetrahydro-1-naphthol,
- 1-Hydroxy-4-methoxy-5,5,8,8-tetramethyl-5,6,7,8-tetrahydro-naphthalincarbonsäure,

- 1,4-Dihydroxy-5,5,8,8-tetramethyl-5,6,7,8-tetrahydronaphthalin,
- 2-Benzoyl-4-methoxy-5,5,8,8-tetramethyl-5,6,7,8-tetrahydro-l-naphthol und
- 2-Benzyl-4-methoxy-5,5,8,8-tetramethyl-5,6,7,8-tetrahydro-1-naphthol.

**9.** Verfahren zur Herstellung von Verbindungen nach den Ansprüchen 1 bis 8, dadurch gekennzeichnet, daß man in einem organischen Lösungsmittel in Gegenwart einer Lewis-Säure ein 2,5-Dichloralkan der Formel:

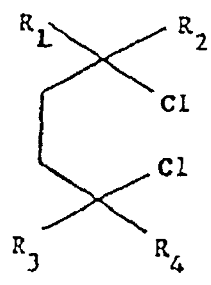

in der
$R_1$, $R_2$, $R_3$ und $R_4$ einen Niedrigalkylrest bedeuten, und ein 4-Alkoxyphenol oder ein 1,4-Dialkoxybenzol der Formel:

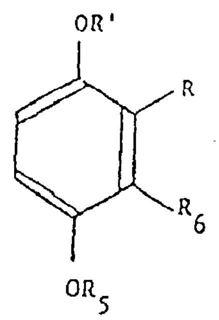

zur Umsetzung bringt, in der
R, $R_5$ und $R_6$ die gleiche Bedeutung wie in Anspruch 1 haben, und R' ein Wasserstoffatom oder einen Niedrigalkylrest bedeutet, und daß man das erhaltene 1,4-Dialkoxy-Derivat (wenn R' einen Alkylrest bedeutet) einer Behandlung in Gegenwart von Jodwasserstoff- oder Bromwasserstoffsäure unterwirft.

**10.** Verfahren nach Anspruch 9, dadurch gekennzeichnet, daß das organische Lösungsmittel 1,2-Dichlorethan und die Lewis-Säure Aluminiumchlorid ist.

**11.** Kosmetische Zubereitung, dadurch gekennzeichnet, daß sie eine wirksame Menge mindestens einer Verbindung der Formel (I) gemäß einem der Ansprüche 1 bis 8 enthält, in einem kosmetisch verträglichen Träger, der mindestens eine Fettphase enthält.

**12.** Zubereitung nach Anspruch 11, dadurch gekennzeichnet, daß sie mindestens eine Verbindung aus folgender Gruppe enthält:
- 4-Methoxy-5,5,8,8-tetramethyl-5,6,7,8-tetrahydro-1-naphthol,
- 2-Acetyl-4-methoxy-5,5,8,8-tetramethyl-5,6,7,8-tetrahydro-1-naphthol,
- 2-(1-Hydroxyethyl)-4-methoxy-5,5,8,8-tetramethyl-5,6,7,8-tetrahydro-1-naphthol,
- 2-(2-Hydroxy-2-propyl)-4-methoxy-5,5,8,8-tetramethyl-5,6,7,8-tetrahydro-1-naphthol,
- 2-(2-Propenyl)-4-methoxy-5,5,8,8-tetramethyl-5,6,7,8-tetrahydro-1-naphthol,
- 1-Hydroxy-4-methoxy-5,5,8,S-tetramethyl-5,6,7,8-tetrahydro-naphthalincarbonsäure,
- 1,4-Dihydroxy-5,5,8,8-tetramethyl-5,6,7,8-tetrahydronaphthalin,
- 2-Benzoyl-4-methoxy-5,5,8,8-tetrameLhyl-5,6,7,8-tetrahydro-1-naphthol, und
- 2-Benzyl-4-methoxy-5,5,8,8-tetramethyl-5,6,7,8-tetrahydro-1-naphthol.

**13.** Zubereitung nach einem der Ansprüche 11 und 12, dadurch gekennzeichnet, daß sie die verbindung der Formel (I) in einem Verhältnis zwischen 0,05 und 10 Gew.-%, bezogen auf das Gesamtgewicht der Zubereitung, enthält.

**14.** Verfahren zum Schutz einer kosmetischen Zubereitung gegen Oxidation, dadurch gekennzeichnet, daß man der Zubereitung eine wirksame Menge mindestens einer Verbindung der Formel (I) einverleibt.

**15.** Pharmazeutische Zubereitung, dadurch gekennzeichnet, daß sie eine therapeutisch wirksame Menge mindestens einer Verbindung der Formel (I) nach den Ansprüchen 1 bis 8 in einem pharmazeutischen Träger oder Exzipienten enthält.

**16.** Pharmazeutische Zubereitung nach Anspruch 15, dadurch gekennzeichnet, daß sie die aktive Verbindung der Formel (I) in einem Verhältnis zwischen 0,01 und 20 Gew.-%, vorzugsweise zwischen 0,1 und 5 Gew.-%, bezogen auf das Gesamtgewicht der Zubereitung, enthält.

**17.** Pharmazeutische Zubereitung nach einem der Ansprüche 15 und 16, dadurch gekennzeichnet, daß die aktive Verbindung der Formel (I) aus folgender Gruppe ausgewählt ist:
- 4-Methoxy-5,5,8,8-tetramethyl-5,6,7,8-tetrahydro-1-naphthol,
- 2-Acetyl-4-methoxy-5,5,8,8-tetramethyl-5,6,7,8-tetrahydro-1-naphthol,
- 2-(1-Hydroxyethyl)-4-methoxy-5,5,8,8-tetramethyl-5,6,7,8-tetrahydro-1-naphthol,
- 2-(2-Hydroxy-2-propyl)-4-methoxy-5,5,8,8-tetramethyl-5,6,7,8-tetrahydro-1-naphthol,
- 2-(2-Propenyl)-4-methoxy-5,5,8,8-tetramethyl-5,6,7,8-tetrahydro-1-naphthol,
- 1-Hydroxy-4-methoxy-5,5,8,8-tetramethyl-5,6,7,8-tetrahydro-naphthalincarbonsäure,
- 1,4-Dihydroxy-5,5,8,8-tetramethyl-5,6,7,8-tetrahydronaphthalin,
- 2-Benzoyl-4-methoxy-5,5,8,8-tetramethyl-5,6,7,8-tetrahydro-1-naphthol und
- 2-Benzyl-4-methoxy-5,5,8,8-tetramethyl-5,6,7,8-tetrahydro-1-naphthol.

**18.** Verwendung einer Verbindung der Formel (I) nach den Ansprüchen 1 bis 8 zur Herstellung einer pharmazeutischen Zubereitung für die präventive Behandlung von Entzündungen und Allergien der Haut.

**19.** Verwendung einer Verbindung der Formel (I) nach den Ansprüchen 1 bis 8 zur Herstellung einer pharmazeutischen Zubereitung für die Prävention bestimmter Krebsarten.

## Claims

**1.** 5,6,7,8-Tetrahydro-1-naphtol derivatives corresponding to the following general formula:

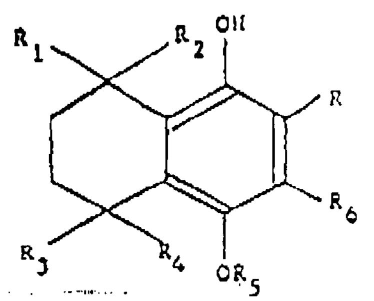

(I)

in which

$R_1$, $R_2$, $R_3$ and $R_4$ denote a lower alkyl radical,

$R_5$ and $R_6$ denote a hydrogen atom or a lower alkyl radical,

R denotes a hydrogen atom, a $C_1$-$C_{18}$ alkyl radical, a $C_2$-$C_{18}$ alkyl radical substituted by one or more hydroxyl(s), a $C_3$-$C_{18}$ alkenyl radical, a $C_2$-$C_{18}$ acyl radical, a benzyl radical, a benzoyl radical, a carboxyl radical and the carboxylic salts of an alkali or alkaline-earth metal or of an organic amine.

**2.** Compounds according to Claim 1, characterised in that the lower alkyl radicals and those which have up to 18 carbon atoms are taken from the group consisting of methyl, ethyl, isopropyl, isobutyl, butyl, tert-butyl, 2-ethylhexyl, isooctyl, dodecyl, hexadecyl and octadecyl radicals.

**3.** Compounds according to Claim 1, characterised in that the $C_2$-$C_{18}$ alkyl radicals substituted by one or more hydroxyl(s) are taken from the group consisting of 1-hydroxyethyl, 2-hydroxypropyl, 1-hydroxypropyl, 1-hydroxybutyl, 1-hydroxyhexyl, 1-hydroxy-1-ethylhexyl or 2,3-dihydroxypropyl radicals.

**4.** Compounds according to Claim 1, characterised in that the alkenyl radicals are taken from the group consisting of propenyl, butenyl, hexenyl, octenyl, dodecenyl and octadecenyl radicals.

**5.** Compounds according to Claim 1, characterised in that the acyl radicals are taken from the group consisting of acetyl, propionyl, butyryl, isobutyryl, hexanoyl, octanoyl, dodecanoyl and octadecanoyl radicals.

**6.** Compounds according to Claim 1, characterised in that, when R is a benzyl or benzoyl radical, the latter can be denoted by the following general formula:

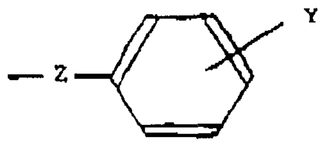

in which:

Z denotes -CO-, -CHOH- or $-CH_2-$ and

Y denotes a hydrogen atom, a halogen, preferably chlorine, an alkoxy, preferably methoxy, or the trifluoromethyl radical.

**7.** Compounds according to Claim 1, characterised in that they are in the form of a sodium, potassium, magnesium or triethanolamine salt when R = -COOH.

**8.** Compounds according to any one of Claims 1 to 7, characterised in that they are taken from the group consisting of:

- 4-methoxy-5,5,8,8-tetramethyl-5,6,7,8-tetrahydro-1-naphthol,
- 2-acetyl-4-methoxy-5,5,8,8-tetramethyl-5,6,7,8-tetrahydro-1-naphthol,
- 2-(1-hydroxyethyl)-4-methoxy-5,5,8,8-tetramethyl-5,6,7,8-tetrahydro-1-naphthol,
- 2-(2-hydroxy-2-propyl)-4-methoxy-5,5,8,8-tetramethyl-5,6,7,8-tetrahydro-1-naphthol,
- 2-(2-propenyl)-4-methoxy-5,5,8,8-tetramethyl-5,6,7,8-tetrahydro-1-naphthol,
- 1-hydroxy-4-methoxy-5,5,8,8-tetramethyl-5,6,7,8-tetrahydronaphthoic acid,
- 1,4-dihydroxy-5,5,8,8-tetramethyl-5,6,7,8-tetrahydronaphthalene,
- 2-benzoyl-4-methoxy-5,5,8,8-tetramethyl-5,6,7,8-tetrahydro-1-naphthol and
- 2-benzyl-4-methoxy-5,5,8,8-tetramethyl-5,6,7,8-tetrahydro-1-naphthol.

**9.** Process for the preparation of the compounds according to Claims 1 to 8, characterised in that it consists in reacting, in an organic solvent in the presence of a Lewis acid, a 2,5-dichloroalkane of formula:

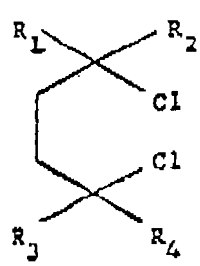

in which:

$R_1$, $R_2$, $R_3$ and $R_4$ denote a lower alkyl radical, with a 4-alkoxyphenol or a 1,4-dialkoxybenzene of formula:

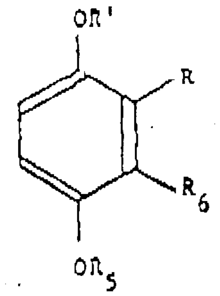

in which:

R, $R_5$ and $R_6$ have the same meanings as those given in Claim 1, and R' denotes a hydrogen atom or a lower alkyl radical, and in that the 1,4-dialkoxy derivative obtained (when R' denotes an alkyl radical) is subjected to a treatment in the presence of hydriodic or hydrobromic acid.

**10.** Process according to Claim 9, characterised in that the organic solvent is 1,2-dichloroethane and the Lewis acid is aluminium chloride.

**11.** Cosmetic composition characterised in that it contains an effective quantity of at least one compound of formula (I) according to any one of Claims 1 to 8 in a cosmetically acceptable carrier containing at least one fatty phase.

**12.** Composition according to Claim 11, characterised in that it contains at least one compound chosen from the group consisting of:
- 4-methoxy-5,5,8,8-tetramethyl-5,6,7,8-tetrahydro-1-naphthol,
- 2-acetyl-4-methoxy-5,5,8,8-tetramethyl-5,6,7,8-tetrahydro-1-naphthol,
- 2-(1-hydroxyethyl)-4-methoxy-5,5,8,8-tetramethyl-5,6,7,8-tetrahydro-1-naphthol,
- 2-(2-hydroxy-2-propyl)-4-methoxy-5,5,8,8-tetramethyl-5,6,7,8-tetrahydro-1-naphthol,
- 2-(2-propenyl)-4-methoxy-5,5,8,8-tetramethyl-5,6,7,8-tetrahydro-1-naphthol,
- 1-hydroxy-4-methoxy-5,5,8,8-tetramethyl-5,6,7,8-tetrahydronaphthoic acid,
- 1,4-dihydroxy-5,5,8,8-tetramethyl-5,6,7,8-tetrahydronaphthalene,
- 2-benzoyl-4-methoxy-5,5,8,8-tetramethyl-5,6,7,8-tetrahydro-1-naphthol and
- 2-benzyl-4-methoxy-5,5,8,8-tetramethyl-5,6,7,8-tetrahydro-1-naphthol.

**13.** Composition according to one of Claims 11 and 12, characterised in that it contains the compound of formula (I) in a proportion of between 0.05 and 10 % by weight relative to the total weight of the composition.

**14.** Process for protecting a cosmetic composition against oxidation, characterised in that it consists in incorporating in this composition an effective quantity of at least one compound of formula (I).

**15.** Pharmaceutical composition characterised in that it contains a therapeutically active quantity of at least one compound of formula (I) according to Claims 1 to 8 in a pharmaceutical carrier or excipient.

**16.** Pharmaceutical composition according to Claim 15, characterised in that it contains the active compound of formula (I) in a proportion of between 0.01 and 20 % and preferably between 0.1 and 5 % by weight relative to the total weight of the composition.

**17.** Pharmaceutical composition according to one of Claims 15 and 16, characterised in that the active compound of formula (I) is chosen from the group consisting of:
- 4-methoxy-5,5,8,8-tetramethyl-5,6,7,8-tetrahydro-1-naphthol,
- 2-acetyl-4-methoxy-5,5,8,8-tetramethyl-5,6,7,8-tetrahydro-1-naphthol,
- 2-(1-hydroxyethyl)-4-methoxy-5,5,8,8-tetramethyl-5,6,7,8-tetrahydro-1-naphthol,
- 2-(2-hydroxy-2-propyl)-4-methoxy-5,5,8,8-tetramethyl-5,6,7,8-tetrahydro-1-naphthol,
- 2-(2-propenyl)-4-methoxy-5,5,8,8-tetramethyl-5,6,7,8-tetrahydro-1-naphthol,
- 1-hydroxy-4-methoxy-5,5,8,8-tetramethyl-5,6,7,8-tetrahydronaphthoic acid,
- 1,4-dihydroxy-5,5,8,8-tetramethyl-5,6,7,8-tetrahydronaphthalene,
- 2-benzoyl-4-methoxy-5,5,8,8-tetramethyl-5,6,7,8-tetrahydro-1-naphthol and
- 2-benzyl-4-methoxy-5,5,8,8-tetramethyl-5,6,7,8-tetrahydro-1-naphthol.

18. Use of a compound of formula (I) according to Claims 1 to 8 for the preparation of a pharmaceutical composition intended for the preventive treatment of cutaneous inflammations and allergies.

19. Use of a compound of formula (I) according to Claims 1 to 8 for the preparation of a pharmaceutical composition intended for the prevention of certain cancers.